# EUROPEAN PATENT SPECIFICATION

(11) **EP 1 796 594 B1**
(45) Date of publication and mention of the grant of the patent: **18.09.2019**
(21) Application number: 05787820.9
(22) Date of filing: 15.08.2005
(51) Int. Cl.: A61F 2/08

(54) **SURGICALLY IMPLANTABLE KNEE PROSTHESIS**
CHIRURGISCH IMPLANTIERBARE KNIEPROTHESE
PROTHESE DE GENOU DESTINEE A UNE IMPLANTATION CHIRURGICALE

(30) Priority: 15.09.2004 US 941729
(43) Date of publication of application: 20.06.2007
(73) Proprietor: Fell, Barry M., Hummelstown, PA 17036 (US)
(72) Inventor: Fell, Barry M., Hummelstown, PA 17036 (US)
(74) Representative: Miller, James Lionel Woolverton
(86) International application number: PCT/US2005/029195
(87) International publication number: WO 2006/036352

(56) References cited:
- WO-A-03/061522
- GB-A- 2 291 355
- US-A- 4 034 418
- US-A- 5 935 173
- US-A1- 2003 060 884
- US-B1- 6 342 075

## Description

### BACKGROUND OF THE INVENTION

### 1. Field of the Invention

The present invention pertains to a prosthetic device which is surgically implantable into a body joint, and more particularly to a knee joint prosthesis which may be surgically implanted between the femoral condyle and tibial plateau of the knee compartment.

### 2. Background Art

Articular cartilage and meniscal cartilage provide the mobile weight bearing surfaces of the knee joint. Damage to these surfaces is generally due to genetic predisposition, trauma, and/or aging. The result is usually the development of chondromalacia, thinning and softening of the articular cartilage, and degenerative tearing of the meniscal cartilage. Various methods of treatment are available to treat these disease processes. Each option usually has specific indications and is accompanied by a list of benefits and deficiencies that may be compared to other options.

The healthy knee joint has a balanced amount of joint cartilage across the four surfaces of this bicompartmental joint (medial femoral condyle, medial tibial plateau, lateral femoral condyle, and lateral tibial plateau). In patients with osteoarthritis, the degenerative process typically leads to an asymmetric wear pattern that leaves one compartment with significantly less articular cartilage covering the weight bearing areas of the tibia and femur than the other compartment. Most commonly, the medial compartment of the knee joint is affected more than the lateral compartment.

As the disease progresses, large amounts of articular cartilage are worn away. Due to the asymmetric nature of the erosion, the alignment of the mechanical axis of rotation of the femur relative to the tibia becomes tilted down towards the compartment which is suffering the majority of the erosion. The result is a varus (bow-legged) deformity in the case of medial compartment disease predominance, or a valgus (knock-kneed) deformity in the case of lateral compartment disease predominance. Factors such as excessive body weight, previous traumatic injury, knee instability, the absence of the meniscus, and genetic predisposition all affect the rate of the disease.

Osteoarthritis is usually defined in stages of Grade I through V, with Grade III revealing significant articular cartilage loss, Grade IV revealing some eburnation of the subchondral bone, and Grade V detailing both significant articular loss and bone loss. The disease manifests itself as periodic to continuous pain that can be quite uncomfortable for the patient. The cause of this pain is subject to many opinions but it is apparent that, as the joint compartment collapses, the collateral ligament on the side of the predominant disease becomes increasingly slack and the tibial and femoral axes move, for example, from a varus to a valgus condition. This increases the stress on the opposing collateral ligament as well as the cruciate ligaments, and shifts the load bearing function of this bicompartmental joint increasingly towards the diseased side. This increasing joint laxity is suspected of causing some of the pain one feels. In addition, as the bearing loads are shifted, the body responds to the increased loading on the diseased compartment with an increased production of bony surface area (osteophytes) in an attempt to reduce the area unit loading. All of this shifting of the knee component geometry causes a misalignment of the mechanical axis of the joint. This misalignment causes an increase in the rate of degenerative change to the diseased joint surfaces, causing an ever-increasing amount of cartilage debris to build up in the joint, and further causing joint inflammation and subsequent pain.

Currently, there is a void in options used to treat the relatively young patient with moderate to severe chondromalacia involving mainly one compartment of the knee. Current treatments include NSAIDS, cortisone injections, hyaluronic acid (HA) injections, and arthroscopic debridement. Some patients cannot tolerate or do not want the risk of potential side effects of NSAIDS. Repeated cortisone injections actually weaken articular cartilage after a long period of time. HA has shown promising results, but is only a short term solution for pain. Arthroscopic debridement alone frequently does not provide long lasting relief of symptoms.

Unfortunately, the lack of long term success of these treatments leads to more invasive treatment methods. Osteochondral allografts and microfracture techniques are indicated for small cartilage defects that are typically the result of trauma. These procedures are not suitable for addressing large areas of degeneration. In addition, osteochondral allografts can only be used to treat defects on the femoral condyle, as tibial degeneration cannot be addressed with this technique. High tibial osteotomy (HTO) corrects the varus malalignment between the tibia and the femur but, because it is performed below the joint line, it does not fill the cartilage void or re-tension the medial collateral ligament (MCL). Removing bone and changing the joint line does not complicate the conversion to total knee arthroscopy (TKA). However, an HTO does leave a hard sclerotic region of bone which is difficult to penetrate, making conversion to a total knee replacement technically challenging.

Currently, patients with large tibial defects require replacement of the existing surfaces with materials other than articular cartilage, namely with uni-condylar (UKR) or total (TKR) knee replacements. Unfortunately, these procedures resect significant amounts of bone (typically 7-9 mm), and primary (first arthroplasty performed on the joint) procedures have a functional life span of only 5-10 years, such that younger patients will likely require revision surgery as they age. Furthermore, the amount of bone loss that is inherent in a UKR or TKR makes a revision (secondary) procedure much more difficult in the future as even more bone must be removed. Revision knee replacement surgery is usually extensive and results in predictably diminished mechanical life expectancy. Therefore, it is best to delay these types of bone resecting surgeries as long as possible.

The only true solution is to rebuild the defective joint by "filling" the joint space with more articular bearing material through a complete resurfacing of the existing femoral condyle and tibial plateau. By replacing the original cartilage to its pre-diseased depth, the joint mechanical axis alignment is restored to its original condition. Unfortunately, these natural articular materials and surgical technology required to accomplish this replacement task do not yet exist. The alternative method is to fill the joint space with a spacer that replaces the missing articular materials.

Attaching a new bearing surface to the femoral condyle is technically challenging and was first attempted, with limited success, over 40 years ago with the MGH (Massachusetts General Hospital) knee. Like a dental crown, it covered both the femoral condyles with Vitallium (CoCr) and would bear against the existing tibial plateau. Tibial covering devices such as the McKeever, Macintosh, and Townley tibial tray maintained the existing femoral surface as the bearing surface but, like the MGH knee, all required significant bone resection, thus making them less than ideal solutions as well. These devices also made no particular attempt to match the patient's specific femoral or tibial geometry, thus reducing their chances for optimal success. Because these devices were made of CoCr, which has different viscoelastic and wear properties from the natural articular materials, any surface geometry which did not closely match the bearing surface of the tibia or femur could cause premature wear of the remaining cartilage due to asymmetric loading.

Newer materials technologies include filling the joint space by injecting polyurethane (U.S. Patent No. 5,795,353) into the joint and anchoring it with holes drilled into the tibial plateau. Others include a series of polymeric materials such as PVA hydrogels in a titanium mesh (see Chang et al, Journal of Biomedical Materials Research 37, 51-59, 1997), biodegradable anhydride prepolymers that can be cross-linked with irradiation by UV light (U.S. Patent No. 5,902, 599), and in vivo grown articular chondrocytes in a collagen fiber or other biocompatible scaffold (U.S. Patent No. 5,158,574). Other low surface energy materials, such as low temperature isotropic (LTI) pyrolitic carbon, have been investigated as bearing surfaces as well. However, these techniques are limited by one's ability to first of all fashion these materials in a conformal manner to replicate the existing knee geometry, while at the same time maintaining their location within the j oint, while further being able to survive the mechanical loading conditions of the knee.

U.S. Patent Nos. 6,206,927 and 6,558,421 and copending U.S. application Serial No. 10/232, 608, disclose a prosthesis for the knee compartment which fills the joint space in order to replace the missing articular materials. This prosthesis provides an anatomically correct bearing surface for both the tibial plateau and femoral condyle to articulate against. Additionally, the prosthesis reduces the concentrated loads on the femoral condyle and its articular cartilage and maintains proper spatial location of the femoral condyle to the tibial plateau, thereby restoring normal joint alignment. Advantageously, the prosthesis does not require any bone resection or any means of bone fixation.

The above-described prosthesis has a conformal femoral surface which is intended to closely approximate the surface shape of a healthy femoral condyle, thus maximizing the load-bearing surface area and reducing the point loading on the condyle. Furthermore, since the prosthesis is not attached to the bone but rather moves, remaining centered with the condyle, throughout its range of motion (ROM), the prosthesis relies on its conformity to the femoral condyle to restore its position if displaced during knee flexion and extension.

Such femoral conformity requires that motion of the prosthesis be allowed as the knee joint flexes, as the position of the femoral condyle on the tibial plateau varies throughout the ROM. The limits of travel of the prosthesis are typically determined by the geometry of both the femoral condyle and the tibial plateau as well as the relative tension of the ligamentous structures. Normal motion of the prosthesis is generally 2-3 mm of anterior-posterior translation with about 20- 40° of external-internal rotation. However, motion of the prosthesis can be exaggerated when there is significant disease in the distal femoral condyle and/or ligament insufficiency. Excessive motion of the prosthesis can cause increased wear on the remaining articular surfaces, leading to increased ligament laxity.

Therefore, in addition to the benefits described above, it is also desired to provide a unicompartmental prosthesis which reduces any excessive motion of the prosthesis throughout the ROM.

WO 03/061522 discloses a knee implant having a concave top surface and convex bottom configuration. US 5,935,173A discloses a knee prosthesis.

### SUMMARY OF THE INVENTION

The present invention is set out in Claim 1.

According to the invention, a unicompartmental prosthesis for implantation into a knee joint compartment between a femoral condyle and its corresponding tibial plateau is provided for reducing any excessive motion. The prosthesis includes a body having a generally elliptical shape in plan and includes a bottom surface and an opposed top surface, where the top surface has a first portion that is generally flat.

According to the invention, the first portion is centrally located on the top surface and is generally elliptical in shape. The top surface can include a second, generally concave portion, where the second portion at least partially surrounds the first portion. In one embodiment, the bottom surface is generally flat. The top surface and the bottom surface are contoured such that the prosthesis is self-centering within the knee joint compartment. A section across the body from the first end to the second end generally has the shape of a negative meniscus, with a periphery of the body being on average of greater thickness than a central portion, of the body. Outside edges along a periphery of the body are preferably rounded. The bottom surface can include a keel extending therefrom, and the body can include an aperture provided therein.

The body can be constructed of a biocompatible material selected from the group consisting of ceramics, metals, metal alloys, hydrogels, reinforced and non-reinforced thermoset or thermoplastic polymers, or composites thereof. The body can include at least one portion made of a relatively low modulus material, such
as reinforced and non-reinforced elastomeric polymers, viscous-elastic materials, and hydrogels. Furthermore, the body can include a material capable of containing living cells, a surface coating applied to the body, or an active material can be associated with the body.

The body further includes a peripheral edge extending between the top and bottom surfaces and having a first side, a second side opposite the first side, a first end, and a second end opposite the first end. According to one embodiment, one of the first and second sides includes an indentation therein, and the first portion can be adjacent the indentation. A first dimension D is defined by the first end and the second end, and a second dimension F is defined by the first side and the second side, where the dimension F is from about 0.25D to about 1.5D. A length of the first portion is from about O.5D to about 0.8D, and a width of the first portion is between about 0.4F and 0.7F.

In one construction, a prosthesis is provided for implantation into a body joint. The body has a generally elliptical shape in plan and includes a bottom surface and an opposed top surface, the top surface having a first, centrally located portion that is generally flat and a second, generally concave portion at least partially surrounding the first portion.

In another construction, a prosthesis for implantation into a knee joint compartment between a femoral condyle and its corresponding tibial plateau. The prosthesis includes a body having a generally elliptical shape in plan. The body has a bottom surface and an opposed top surface, the top surface having a first portion that is generally flat. The body includes a material capable of spanning any defects in the femoral condyle and tibial plateau without substantially deforming into the defects.

Correspondingly, a method for implantation of a prosthesis into a knee joint compartment between a femoral condyle and its corresponding tibial plateau is provided. While the implantation methods described herein do not form part of the invention, they are disclosed as they represent useful background for understanding the invention. The method includes providing a prosthesis including a hard body having a generally elliptical shape in plan and having a bottom surface and an opposed top surface, the top surface having a first portion that is generally flat. The body further
includes surgically exposing the knee joint compartment and inserting the prosthesis into the knee joint compartment.

The method can further include providing an active material associated with the body or applying a surface coating to the body. In addition, the method can include altering the condition of tissue located in the knee joint compartment. Still further, the method can include determining a size and shape of the prosthesis required by examination of the knee joint, where the examination includes one or more of X-ray imaging and MRI imaging. The prosthesis can be selected from a library of prostheses of standard shapes and sizes, or a custom prosthesis can be generated whose size and shape are at least partially based on the examination of the knee joint. The method can also include providing a keel on the bottom surface, or providing an aperture in the body for use in restraining movement of the prosthesis.

Furthermore, a method is provided for correcting misalignment in an axis of rotation of a knee joint. The method includes providing a prosthesis including a hard body having a generally elliptical shape in plan and having a bottom surface and an opposed top surface, the top surface having a first portion that is generally flat. The method further includes surgically exposing the knee joint, and inserting the prosthesis into the knee joint to at least partially correct the misalignment of the axis of rotation. The method can include inserting the prosthesis includes inserting the prosthesis into a medial compartment of the knee joint and moving the axis to a less varus condition, or can include inserting the prosthesis includes inserting the prosthesis into a lateral compartment of the knee joint and moving the axis to a less valgus condition.

The above features and advantages, along with other features and advantages of the present invention are readily apparent from the following detailed description of the invention when taken in connection with the accompanying drawings.

### BRIEF DESCRIPTION OF THE DRAWINGS

FIGURE 1 is a perspective view illustrating an implantable knee prosthesis according to the present invention;
FIGURE 2 is a top plan view of the prosthesis of FIG. 1;
FIGURE 3 is a side elevational view of the prosthesis of FIG. 1;
FIGURE 4 is an end elevational view of the prosthesis of FIG. 1;
FIGURE 5 is a cross-sectional view of the prosthesis of FIGS. 1-4 taken along line A-A of FIG. 2;
FIGURE 6 is a cross-sectional view of the prosthesis of FIGS. 1-4 taken along line B-B of FIG. 2;
FIGURE 7 is a top plan view of a prosthesis including a keel according to an example of the present invention;
FIGURE 8 is a top plan of a prosthesis is including apertures according to another example of the present invention;
FIGURE 9 is a top plan view of the prosthesis according to the present invention with reference to a coordinate system; and
FIGURE 10 illustrates an exemplary placement of the prosthesis according to the present invention in a knee joint.

### DETAILED DESCRIPTION OF EMBODIMENTS OF THE PRESENT INVENTION

The prosthesis according to the present invention is designed to be surgically implantable into a body joint to replace damaged tissue therein. More particularly, the prosthesis of the present invention is a unicompartmental device suitable for minimally invasive, surgical implantation into a knee compartment requiring little or no bone resection. The knee compartment is defined by the space between a femoral condyle and the respective tibial plateau, in which a portion of the natural meniscus is ordinarily located. By effectively replacing worn articular material, the prosthesis of the present invention restores the normal joint alignment and provides a smooth bearing surface against which the femoral condyle can articulate. Degeneration of the femoral anatomy is significantly reduced because the conforming femoral surface of the prosthesis accommodates the complex shape of the femoral condyle in extension as well as in flexion. Further, it essentially eliminates articulation of the femoral condyle against the tibial plateau, thereby preventing further degradation of the tibial surface. By occupying the joint space and retensioning the collateral ligaments, the prosthesis according to the present invention improves joint stability and restores the limb to a more normal mechanical alignment.

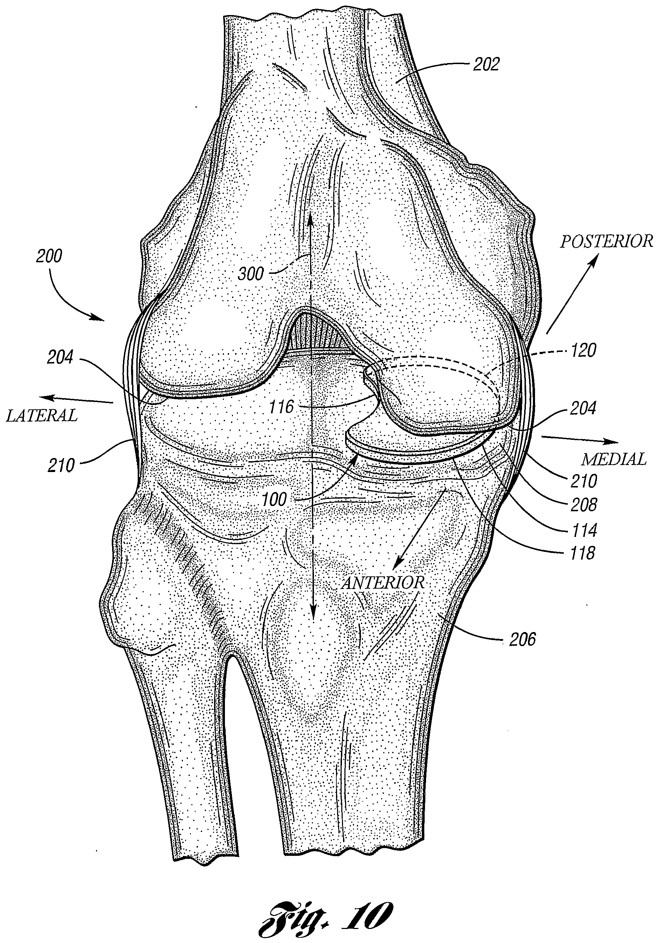

By the term "unicompartmental" it is meant that each prosthesis according to the present invention is suitable for implantation into but one medial or lateral compartment defined by the space between a femoral condyle and its associated tibial plateau. In other words, the present prosthesis is not a bicompartmental prosthesis which, in one rigid device, could be inserted into both of the two femoral condyle/tibial plateau compartments. In many, if not most, cases a prosthesis will be inserted into one compartment only, either the medial or lateral compartment. Most often, it will be the medial compartment as the meniscus and associated articular surfaces in the medial compartment are most subject to wear and damage. Additionally, it is possible to insert two separate prostheses into the medial and lateral compartments of the same knee, or to use two such prostheses that are mechanically, but non-rigidly, linked. Advantageously, the prosthesis according to the present invention functions to at least partially correct misalignment in the knee axis of rotation due to disease. More specifically, when placed in the medial compartment, the prosthesis moves the knee axis 300 (see FIG. 10) into a less varus, more valgus condition (typically 0-5° valgus). Likewise, when placed in the lateral compartment, the prosthesis moves the knee axis 300 into a less valgus condition.

The prosthesis according to the present invention is can be translatable but self-centering. By "translatable" it is meant that during natural articulation of the knee joint the prosthesis is allowed to move or change its position, such that articulation of the knee results in a modest amount of lateral/medial and anterior/posterior translation of the prosthesis relative to the tibial plateau.

The femoral condyle has two major anterior/posterior radii such that, when the knee is in full extension, one radius position is in contact with the tibial plateau while, during flexion, another portion of the femoral condyle is in contact with the tibial plateau. In addition, the femur rotates with respect to the tibia during flexion, thereby changing the orientation of the femoral anatomy to the tibial plateau. The term "self-centering" means that upon translation from a first position to a second position during knee articulation, the prosthesis of the present invention will return to substantially its original position as the articulation of the knee joint is reversed and the original knee position is reached. Thus, the prosthesis will not progressively "creep" towards one side of the compartment in which it is located, but rather the prosthesis is shaped such that the contour of the prosthesis and the natural articulation of the knee exerts a restoring force on the free-floating prosthesis. The angle of attack of the femoral condyle and/or tibial plateau bearing surfaces against the prosthesis will ensure that the prosthesis reversibly translates during articulation, maintaining the prosthesis, on average, in the same location for any given degree of knee articulation. The centered, rest position, of the prosthesis is usually determined when the knee is in extension and there is maximum contact between the femoral condyle and the prosthesis. In order to ensure the ability of the prosthesis to "self-center," adequate tension of the cruciate ligaments should be maintained.

While the prosthesis according to the present invention is shown and described herein as being implanted in a knee joint, it is understood that the prosthesis could be utilized in joints other than the knee, such as the hip, shoulder, wrist, ankle, or elbow.

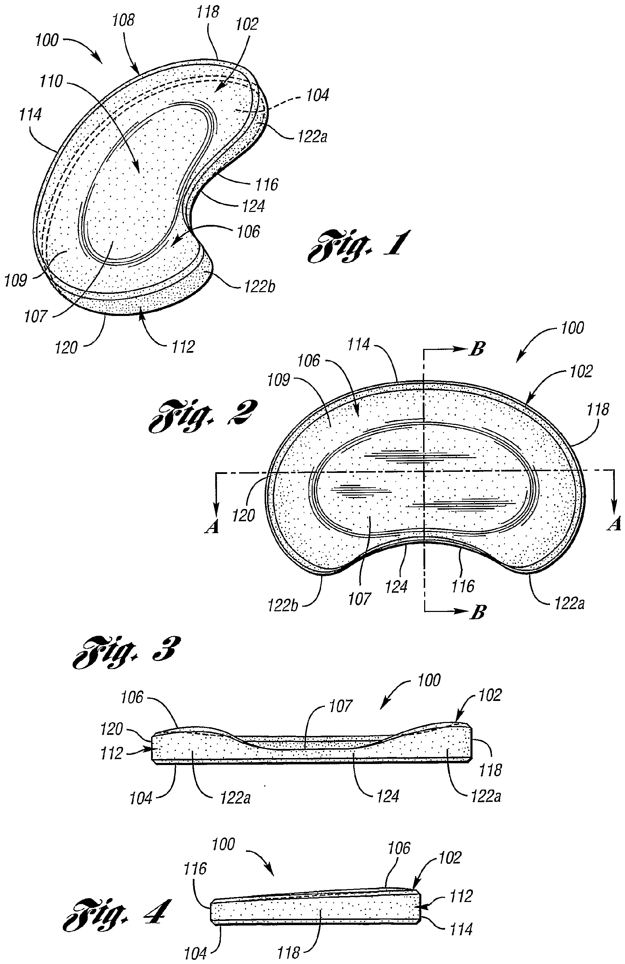

Turning now to FIGS. 1-4, an implantable knee prosthesis according to the present invention is illustrated and designated generally by reference numeral 100. Prosthesis 100 includes a body 102 having a generally elliptical shape in plan and including a bottom, or tibial, surface 104 and an opposite top, or femoral, surface 106. According to one aspect of the present invention, bottom surface 104 is generally flat, wherein the contour of bottom surface 104 is generally the same as the associated contour of the tibial plateau after any necessary bone resection. However it is understood that other contours of bottom surface 104 are fully contemplated in accordance with the present invention. For example, depending on the condition of the ligaments and other soft tissue structure at the time of surgery and how much stability the knee will require, for a medial compartment implantation bottom surface 104 typically ranges from generally flat to convex. For a lateral compartment implantation, bottom surface 104 typically ranges from generally flat to concave. It is understood that the terms "concave" and "convex" as used herein are not restricted to describing surfaces with a constant radius of curvature, but rather are used to denote the general appearance of the surfaces.

Top surface 106 includes a first portion 107 that is generally flat, thereby reducing the conformity of the femoral surface. According to one aspect of the present invention, first portion 107 is centrally located and is generally elliptical in shape. Reducing the conformity of prosthesis 100 in turn reduces the motion of prosthesis 100 during knee extension and flexion, which can advantageously prohibit excessive motion in cases where significant disease exists in the femoral condyle or ligament insufficiency is present. As such, prosthesis 100 according to the present invention prevents increased wear on the remaining articular surfaces as well as increased ligament laxity which can be causes by excessive prosthesis motion during knee flexion and extension. As depicted in FIGS. 1-4, top surface 106 also includes a second portion 109 that at least partially surrounds first portion 107. Second portion 109 is preferably generally concave for a medial compartment implantation, so as to generally conform to the associated contour of the femoral condyle after any necessary bone resection. This conformity allows the prosthesis 100 to remain centered with the femoral condyle throughout its ROM and provides the prosthesis 100 with the ability to effectively restore its original position should it be displaced.

For a lateral compartment implantation, second portion 109 of top surface 106 can range from generally convex to generally concave, depending on the condition of the ligaments and other soft tissue structure at the time of surgery and how much stability the knee will require. Again, it is understood that the terms "concave" and "convex" as used herein is not restricted to describing a surface with a constant radius of curvature, but rather is used to denote the general appearance of the surface.

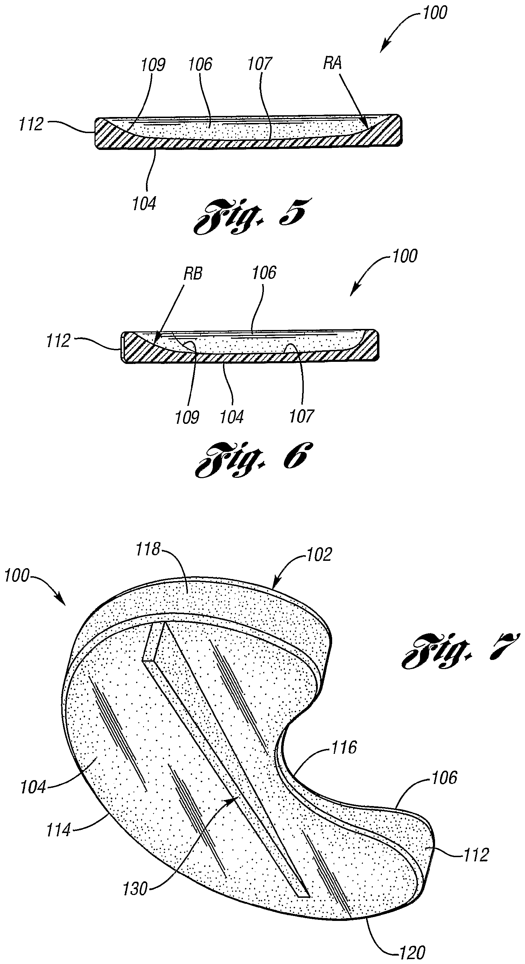

FIG. 5 shows an anterior/posterior cross-sectional view of prosthesis 100 taken along section line A-A of FIG. 2. Similarly, FIG. 6 illustrates a medial/lateral cross-sectional view of prosthesis 100 taken along section line B-B of FIG. 2.

With continued reference to FIGS. 1-4, body 102 further includes a peripheral edge 112 extending between bottom surface 104 and top surface 106 and having a first side 114, a second side 116 opposite first side 114, a first end 118, and a second end 120 opposite first end 118. As shown, edges along the periphery of prosthesis 100 are rounded, which is desired due to the fact that prosthesis 100 can be allowed to move within the joint cavity. Periphery 108 of body 102 is on average of greater thickness than a central portion 110 of body 102 (see FIG. 1), and body 102 generally has a negative meniscus shape when viewed from the side (see FIG. 3) or in a section across body 102 in an anterior-posterior direction from first end 118 to second end 120 (see, for example, FIG. 5).

As depicted herein, second side 116 of body 102 includes a pair of lobes 122a and 122b, where lobe 122a is adjacent first end 118 and lobe 122b is adjacent second end 120, with an indentation 124 formed therebetween. When implanted in a patient's knee compartment (see FIG. 10), indentation 124 will be proximate to the tibial spine and can preferably be designed to vary in shape from patient to patient as necessary due to the great range of variability of human anatomy. With indentation 124, prosthesis 100 is generally kidney-shaped when viewed in plan, with the shape resembling a distorted ellipse, where first portion 107 is adjacent indentation 124. Of course, indentation 124 is not required, and other variations of body configuration are fully contemplated according to the present invention. Accordingly, it is understood that the term "generally elliptical" is intended to include all construction methods which yield a generally planar shape which is longer in one direction than in the transverse direction and has rounded corners, and that prosthesis 100 is not otherwise limited to any particular shape.

To restrain movement of prosthesis 100, one or more keels 130 can be provided on bottom surface 104 as depicted in FIG. 7. Keel 130 can have any shape, such as the tapered wedge shape depicted herein, and can be oriented as desired. For example, keel 130 can generally extend between first end 118 and second end 120 as shown, between first side 114 and second side 116, or at any angle therebetween. Keel 130 can be useful in maintaining prosthesis 100 positioned on the tibial plateau.

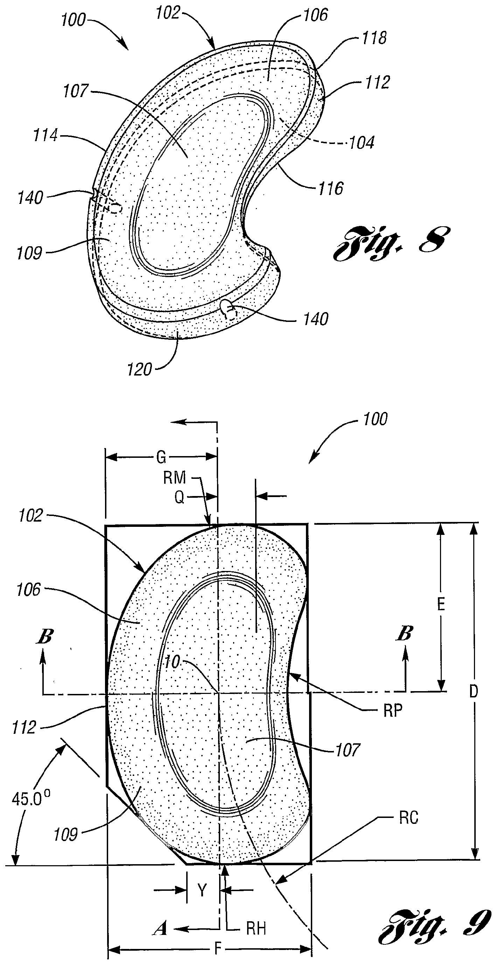

According to another example of the present invention, one or more apertures 140 can extend through body 102 as shown in FIG. 8. Apertures 140 can be at various angles and extend from one of bottom surface 104, top surface 106, or peripheral edge 112 to another of bottom surface 104, top surface 106, or peripheral edge 112. As such, an aperture 140 can extend from bottom surface 104 to either top surface 106 or peripheral edge 112. Also, an aperture 140 can extend from top surface 106 to either bottom surface 104 or peripheral edge 112. Further, an aperture 140 can extend from peripheral edge 112 to either bottom surface 104, top surface 106, or another portion of peripheral edge 112. Apertures 140 can be useful for attachment by a suture or a surgical nail (not shown) to the tibial plateau or surrounding soft tissue. If attached to the soft tissue, motion of prosthesis 100 would be limited to the natural movement of the soft tissue during the range of motion.

Contrary to most devices which are composed of soft, compliant material designed to assume the function of the natural meniscus which they replace, prosthesis 100 of the present invention comprises a relatively hard, relatively high modulus, preferably low friction material. Suitable materials include, for example, metals such as steel or titanium, metal alloys such as those described in U.S. Patent Nos. 3,989,517; 5,368,659; and 5,618,359 (LiquidMetal, Inc.), ceramics, hydrogels, and reinforced and non-reinforced thermoset or thermoplastic polymers. From the hardness desired of prosthesis 100 it is readily apparent that the prosthesis functions in a manner completely different from those of the prior art. It is understood that the term "hard" as used herein is used to describe a material that is sufficient to span defects in the tibia or femur without substantially deforming into the defects, allowing for the provision of recessed or non-contacting areas of the prosthesis to encourage articular regeneration. Since the naturally occurring articular and meniscal materials found in the human knee joint demonstrate and possess unique visco-elastic properties not yet available in man-made material formulations, if such a material were to become available, it would most likely represent the ideal material composition for prosthesis 100.

Prosthesis 100 need not be made only of a single material, but composite structures may be used. In greater detail, materials could include elastomeric polymers such as nylon, silicone, polyurethane, polypropylene, polyester, or the like, optionally fiber-reinforced, or viscous-elastic materials such as hydrogels, as well as other hydrophilic materials or hydrophobic materials. Materials, such as biocompatible polymers, capable of containing living cells could also be utilized. Still other possible materials are those which can replicate the function of naturally occurring cartilage or meniscus such as the CSTI meniscal repair material that is the subject of numerous U.S. patents to Stone, for example, U.S. Patent No. 5,158,574. A surface coating can be employed, such as for the reduction of friction of prosthesis 100. Generally, the areas of prosthesis 100 expected to have the most wear due to either high stress or greater movement relative to the femoral condyle or tibial plateau may be made of stronger, more abrasion resistant material than the remainder of prosthesis 100 when composite structures are used. However, it is understood that any single component may be softer than the material used for constructing the majority of prosthesis 100.

In accordance with the present invention, prosthesis 100 may be manufactured so as to substantially contain, or have deposited thereon, a biologically or pharmaceutically active material such as, for example, one that promotes tissue regrowth, retards tissue degeneration, or decreases inflammation. This is particularly suitable when prosthesis 100 functions to bridge a defective area of bone or articular cartilage. The active material can be provided in the form of a coating on prosthesis 100, or can be contained within prosthesis 100 in the form of a solid, liquid, gel, paste, or soft polymer material. Such active materials may be designed to be delivered at once or in a timed-release manner. Preferably, the area of prosthesis 100 containing the active material does not actually contact, or minimally contacts, the damaged tissue.

As stated above, one purpose of the prosthesis 100 of the present invention is to achieve a span-like effect to bridge areas of the femoral condyle and/or tibial plateau which have been damaged or have experienced tissue degeneration. If too soft and/or low modulus of a material were to be used for the prosthesis as in prior art devices, not only would the load not be concentrated on healthy tissue, but damaged areas would also be subjected to static and dynamic loading and wear, thereby decreasing the opportunity for the body's natural regenerative capability to function. Under such circumstances, active materials will be rapidly dissipated and newly regenerated articular cartilage not having the necessary density or cohesiveness to withstand wear will quickly be eroded away. Rather than substantially deforming as in prior art devices to distribute a load relatively equally on the mating femoral and tibial surfaces, prosthesis 100 according to the present invention does not necessarily spread the load uniformly, but rather may redistribute the load to healthy tissue, spanning areas of imperfection and allowing inflamed, diseased, or other damaged areas to regenerate. Moreover, as regeneration proceeds, the regenerating tissue will assume a shape dictated by the shape of prosthesis 100. Growth under these circumstances has the greatest potential for dense, ordered cartilage most closely replicating the original surface.

Much study has been dedicated to determine if any relationship exists in the normal human anatomy that would allow one to define the required dimensions of the prosthesis for proper fit and function based on a single, easy to establish, measurable anatomic landmark. Based on a study of over 100 MRI's and 75 X-rays of human subjects ranging from 15 to 87 years of age, a relationship was established between the anteroposterior radius of the most distal portion of the femoral condyle and the dimensions which control the geometric form of the prosthesis. The database revealed a range of femoral anteroposterior radii from 32 mm to 48 mm. However, it is known that the worldwide range is much larger because of genetic differences in the human anatomy.

With reference now to FIG. 9, a method of construction of prosthesis 100 according to one example of the present invention aligns the apex of a femoral radius with the Coordinate System Origin (CSO) 10. The apex of a tibial surface is also generally aligned in both the anterior/posterior with the CSO 10, but is separated vertically from the CSO 10 to create the part thickness. The substantially elliptical shape of peripheral edge 112 is then located with respect to the CSO 10. In general, the CSO 10 of the prosthesis 100 is located at the center of the ellipse. It has been found that a suitable size for body 102 is defined by a minor axis of the ellipse F (defined by first side 114 and second side 116) and a major axis D (defined by first end 118 and second end 120) which are related by a ratio ranging from F = 0.25D to 1.5D. A length of the first portion is from about 0.5D to about 0.8D, and a width of the first portion is between about 0.4F and 0.7F.

Similar ratios can be established for all of the controlling dimensions of the part such that the shape in plan, femoral surface geometry, and tibial surface geometry for a normal tibial anatomy can generally be defined by one physical anterior/posterior measurement of the patient's tibial anatomy. The appropriate thickness of the prosthesis 100 can be determined by measuring the amount of joint space between the femoral and tibial surface when a minor amount of valgus (heels out, knees in) is applied to the knee.

Referring to FIGS. 5-6 and 9, the preferred relationship between femoral radius RA (see FIG. 5) to other joint dimensions (femoral radius is the driving relationship to all other dimensions) is as follows:
Medial-lateral radius RB = 0.25RA to 1.0RA (see FIG. 6)
Curve of anterior half of femoral radius RC = 0.5RA to 2.0RA, posterior half is straight
Length D = 0.6RA to 1.4RA
Posterior half E = 0.1RA to 0.75RA
Width F = 0.25RA to 1.5RA
Width from part center to medial edge G = 0.096RA to 0.48RA
Anterior plan radius RH = 0.16RA to 0.64RA
Posterior plan radius RM = 0.16RA to 0.64RA
Radius along lateral spine area RP = 0.1RA to 2.0RA
Width from part center to lateral edge Q = -0.32RA to 0.32RA
Location of transition from anterior radius to medial radius Y = -0.32RA to 0.32RA

(A negative value means that a dimension may extend to an opposite side of section line A-A).

The actual shape of prosthesis 100 may be tailored to the individual. Individuals with high varus or valgus deformation due to wear, degeneration, or disease may require a prosthesis which is of considerably greater thickness over the portions where wear is most advanced. In youthful patients, where trauma-induced damage rather than severe wear or degeneration has occurred, differences in prosthesis thickness will be more moderate.

The axis of rotation of the tibia on the femur is 90 degrees to the path of the tibial plateau against the femoral condyle. The two tibial plateaus (medial and lateral) are not in the same plane with each other but do act in a relatively constant radius to their respective femoral condyles. In other words, although the symmetry of the femoral side of the prosthesis may be matched with the femoral condyle while the leg is in full extension, the rotation of the tibial plateau against the femoral condyle is along a constant axis of rotation (90 degrees to the axis of rotation), thus the angularity of the axis of symmetry of the femoral condyle relative to the axis of symmetry of the tibial plateau is not parallel but at some acute angle. Also, the axis of symmetry of the tibial plateau is not parallel to the path of rotation of the tibia relative to the femur, but also at some mildly acute angle. Thus, the true orientation of the prosthesis, regardless of the relative orientations of symmetry of the tibial side to the femoral side is 90 degrees to the true axis of rotation as described in Hollister et al., "The Axes of Rotation of the Knee," Clin Orthopaedics and Rel Res 290, pp. 259-268, 1993, herein incorporated by reference. Any localized positions of higher loads are self-limiting due to the ability of the prosthesis to translate both rotationally and laterally which mimics the true motion of the natural meniscus as described by Hollister.

During the load bearing portion of the gait cycle, or stance phase, flexion at the knee does not typically exceed 35°. Thus, the highest compressive loads in the knee occur with the knee substantially extended. The outer contours of the prosthesis are preferably designed to substantially mate with the corresponding tibial and femoral surfaces when the knee is in full extension so that the high compressive loads can be distributed over large surface areas. The contact areas between the femoral condyle and the femoral surface of the prosthesis and between the tibial plateau and the tibial surface of the prosthesis are substantially equivalent during extension. However, because the contour of the femoral surface is more concave, the femoral condyle determines the position of the prosthesis on the surface of the tibial plateau in extension.

As the knee is flexed, the mating along the tibial surface is substantially maintained. However, the contoured mating surfaces of the femoral condyle and femoral surfaces of the prosthesis of the present invention can become increasingly dissimilar when the joint articulates. As the knee is flexed, there is a relative external rotation and posterior translation of the femur with respect to the tibia. Thus, the contour angle of the femur becomes more in-line with the contour angle of the tibia in flexion. This can cause relative lateral or rotational movement, in the tibial plane, between the femoral condyle and the femoral surface of the prosthesis. The forces generated by the increasingly different geometry creates a rotational moment in the tibial plane which is resisted along the mating tibial surfaces and which also results in a restoring force tending to correctly locate the prosthesis along the femoral condyle. Thus, the prosthesis is self-centering to the femoral condyle, in part as a result of the conformity between the femoral condyle and the femoral surface of the prosthesis.

By changing the femoral surface of the prosthesis, it is possible to reduce the rotational moment induced during flexion by the mismatch between the femoral surface of the prosthesis and the femoral condyle. One method to accommodate this motion is to have a less acute alignment between the femoral and tibial axes of symmetry posterior to the anterior/posterior midline, thereby reducing the mismatch between the two axes in flexion. This angle is preferably 0° and can range from +/- 15°. Anterior to the midline, the femoral contour is bent around a radius RC that is tangent to the posterior section of the sweep plane at the most distal point of the femoral anterior/posterior radius RA. This femoral surface geometry is essentially a compromise between the different extension and flexion alignments of the femoral and tibial axes of symmetry.

Because prosthesis 100 according to the present invention may be utilized with no physical method of attachment, the femoral surface serves to locate the prosthesis through all ranges of motion, provided that the collateral ligaments are in proper tension. By the very nature of the ability to adjust for the lost articular material through the thickness of the prosthesis, the thickness adjustment substantially eliminates the need for a functional meniscus as a bearing surface in a severely (Grade III or IV) degenerated knee. In these instances, the femoral surface of the prosthesis resides significantly above the meniscal edge, and the meniscus is completely unloaded. Prosthesis 100 according to the present invention also increases the translational stability of the knee, as the conforming shape of the femoral surface plus re-tensioning of the collateral ligaments limits excessive anterior to posterior translation of the femur.

Generally speaking, each knee presents a different geometry of the respective femoral condyles and tibial plateaus. Even with respect to the right and left knees of a single individual, although bilateral symmetry dictates that the left and right knee components should be mirror images, this is often only an approximation. Thus, the shape of the affected femoral condyle and tibial plateau (while discussed herein in the singular, more than one pair of condyle(s)/plateau(s) may be involved), will have to be ascertained to determine the correct geometry of the prosthesis 100 for a given patient.

To implant a prosthesis that possesses the characteristics required by the subject invention, the patient's knee joint may be examined by a non-invasive imaging procedure capable of generating sufficient information such that one appropriately sized and shaped device may be selected. A variety of non-invasive imaging devices may be suitable, for example magnetic resonance imaging (MRI), X-ray devices and the like.

Two methods of non-invasive imaging for selection of a suitable prosthesis 100 are preferred. In the first method, MRI or other non-invasive imaging scans, optionally coupled with exterior measurements of the dimensions of the relevant tibial and femoral portions including the surface of the articular cartilage of the tibia and femur, may be used to establish a library of prostheses whose size and geometry differ according to the age and size of the patient, the patient's genetic make-up, and the like. A limited number of "standard" prostheses are then made to meet the requirements of a generic population of patients. In this first method, a non-invasive imaging scan, such as an X-ray or MRI, together with knowledge of the patient's genetic make-up, general body type, extent of the disease, degeneration, or trauma and the like, will enable the surgeon to select a prosthesis 100 of the correct size and shape from the library for the patient.

In a second method, each patient receives one or more prostheses that are custom tailored for the individual by producing a contour plot of the femoral and tibial mating surfaces and the size of the meniscal cavity. Such a contour plot may be constructed from imaging data (i.e., X-ray or MRI data) by a suitable computer program. From the contour plot, the correct surface geometry of the prosthesis 100 is determined from the shape of the respective tibial plateau and femoral condyle and the orientation between the two surfaces in extension. In general, the shapes just mentioned also include the articular cartilage which is typically maintained substantially intact.

In accordance with the present invention, it has been discovered that the amount of varus deformity is the primary, non-invasive method for determining the necessary thickness of the prosthesis 100 required for proper functioning. Viewing a weight bearing anteroposterior X-ray, a cut and paste of the line drawn through the femoral condyles and repositioned to put them once again parallel to the tibial plateaus will yield a measurement for the approximate thickness of the prosthesis. However, typically the proper thickness of the prosthesis is determined intra-operatively.

Insertion of the prosthesis 100 of the present invention is typically done via a 3 cm to 5 cm medial parapatella incision. The prosthesis 100 is introduced by arthroscopically assisted implantation, generally limited to extensive clean-up of existing damaged tissue, e.g., torn or particulate natural meniscus damage, osteophyte resection, etc. The natural meniscus may be maintained in position or may be wholly or partially removed, depending upon its condition. Under ordinary circumstances, pieces of the natural meniscus which have been torn away are removed, and damaged areas may be trimmed as necessary. In somewhat rarer instances, the entire portion of the meniscus residing in the meniscal cavity may be removed or is not present. No bone resection or mechanical fixation of the prosthesis 100 is required. Only osteophytes which interfere with the prosthesis placement or with proper collateral ligament alignment are removed.

Prosthesis 100 may also be used in conjunction with ACL or PCL repair, tibial osteotomy or articular surfacing procedures such as cartilage transplantations or abrasion anthroplasty. Following insertion of the prosthesis, X-ray, fluoroscopy, or MRI may be used to assess the correct positioning of the prosthesis both intraoperatively as well as postoperatively. Since the surgical procedures used are not severe, and also not irreversible, an unsuitable prosthesis may be readily removed and replaced, either with a different prosthesis from the library, or by a custom prosthesis.

FIG. 10 illustrates prosthesis 100 positioned in a right knee joint 200 between a femur 202, including the femoral condyles 204, and a tibia 206 including the tibial plateau 208. The femur 202 and tibia 206 include interconnecting collateral ligaments 210. FIG. 10 illustrates the position of first side 114, second side 116, first end 118, and second end 120 of prosthesis 100 when inserted in the medial compartment of a patient' right knee joint 200. It is understood that prosthesis 100 may just as easily be implanted in a lateral compartment or in the left knee of a patient.

Following any necessary bone resection, one surgical procedure which may be used to implant prosthesis 100 according to the present invention includes the following steps:
1. Verify preoperative indications:
   a. Valgus determination of < 5° with erect anterior/posterior X-ray;
   b. Medial compartment disease only. Some lateral spurs may be present; and
   c. Pre-operative sizing via medial/lateral template measurement of anterior/posterior X-ray.
2. Standard Arthroscopy surgical prep:
   a. Infiltrate knee with Lidocaine/Marcaine and Epinephrine.
3. Arthroscopy:
   a. Inspect lateral patello-femoral compartments for integrity, some mild arthosis is acceptable;
   b. Removal of medial meniscus toward the rim along the anterior, medial and posterior portions;
   c. Initial arthroscopic osteophyte removal via 1/8" osteotome and burr to allow for valgus positioning of the knee;
   d. Complete the removal (to the rim) of the posterior and posterior-lateral meniscus; and
   e. Confirm sizing of the prosthesis by measuring distance from resected meniscus to remaining anterior meniscus.
4. Medial parapatellar arthrotomy (mid-patella to tibial joint line).
5. Complete removal of visible osteophytes along the medial femoral condyle.
6. Insert thickness gauge and size for thickness of prosthesis.
7. Insert trial component:
   a. Flex knee to approximately 50+ degrees to fully expose the distal portion of the femoral condyle;
   b. Insert trial component; and
   c. While applying insertion pressure, apply valgus stress to the tibia and "stretch-extend" the tibia over the trial component.
8. Check for proper sizing with "true lateral" and anterior/posterior fluoroscope images of the knee while in extension:
   a. Ideally, the prosthesis should be within 1 mm of the anterior/posterior boundaries of the tibial plateau and superimposed over the medial boundary.
9. Remove trial component and flush joint with saline.
10. Insert the appropriate prosthesis.
11. Confirm proper placement and sizing with fluoroscopic images as with trial component.
12. Maintain leg in extension and close wound after insertion of a Hemovac drain.
13. Place leg in immobilizer prior to patient transfer.

While embodiments of the invention have been illustrated and described, it is not intended that these embodiments illustrate and describe all possible forms of the invention. The words used in the specification are words of description rather than limitation, and it is understood that various changes may be made without departing from the scope of the invention.

## Claims

1. A unicompartmental prosthesis (100) for implantation into a knee joint compartment between a femoral condyle and its corresponding tibial plateau, the prosthesis (100) comprising:
a body (102) having a generally elliptical shape in plan, the body (102) including a bottom surface (104) configured with a contour that is generally the same as the tibial plateau and an opposed top surface (106), the body (102) including a peripheral edge (112) extending between the top and bottom surfaces (104, 106) and having a first side (114), a second side (116) opposite the first side (114), a first end (118), and a second end (120) opposite the first end (118), **characterized by** the top surface (106) having a first, centrally located portion (107) that is elliptically shaped and generally flat for reducing conformity of the top surface (106) with the femoral condyle to reduce excessive motion of the body during knee joint articulation due to disease in the femoral condyle or ligament insufficiency and a second, concave portion (109) extending upwardly from the first portion (107) and spaced from the peripheral edge (112) around an entire periphery of the first portion (107), the first portion (107) having a different radius of curvature than the second portion (109), and wherein the second portion is configured to generally conform with a contour of the femoral condyle so as to allow the body to remain centered with the femoral condyle within the knee joint compartment throughout knee articulation.

2. The prosthesis according to claim 1, wherein the bottom surface (104) is generally flat.

3. The prosthesis according to claim 1, wherein the bottom surface (104) includes at least one keel (130) extending therefrom.

4. The prosthesis according to claim 1, wherein the body (102) includes at least one aperture (140) provided therein.

5. The prosthesis according to claim 1, wherein the body (102) is constructed of a biocompatible material selected from the group consisting of ceramics, metals, metal alloys, hydrogels, reinforced and non-reinforced thermoset or thermoplastic polymers, or composites thereof.

6. The prosthesis according to claim 1, wherein the body (102) includes at least one portion made of a relatively low modulus material selected from the group consisting of reinforced and non-reinforced elastomeric polymers, viscous-elastic materials, and hydrogels.

7. The prosthesis according to claim 1, wherein the body (102) includes a material capable of containing living cells.

8. The prosthesis according to claim 1, further comprising a surface coating applied to the body (102).

9. The prosthesis according to claim 1, wherein the body (102) includes an active material associated therewith.

10. The prosthesis according to claim 1, wherein the top surface (106) and the bottom surface (104) are contoured such that the prosthesis (100) is self-centering within the knee joint compartment.

11. The prosthesis according to claim 1, wherein one of the first and second sides (114, 116) includes an indentation (124) therein.

12. The prosthesis according to claim 11, wherein the first portion (107) is adjacent the indentation (124).

13. The prosthesis according to claim 1, wherein a first dimension D is defined by the first end (118) and the second end (120), and a second dimension F is defined by the first side (114) and the second side (116), wherein the dimension F is from about 0.25D to about 1.5D.

14. The prosthesis according to claim 13, wherein a length of the first portion (107) is from about 0.5D to about 0.8D.

15. The prosthesis according to claim 13, wherein a width of the first portion (107) is between about 0.4F and 0.7F.

16. The prosthesis according to claim 1, wherein a section across the body (102) from the first end (118) to the second end (120) generally has the shape of a negative meniscus.

17. The prosthesis according to claim 1, wherein a periphery of the body (102) is on average of greater thickness than a central portion of the body (102).

18. The prosthesis according to claim 1, wherein the body (102) comprises a material capable of spanning any defects in the femoral condyle and tibial plateau without substantially deforming into the defects.

19. The prosthesis according to claim 1, wherein the prosthesis (100) is free of physical attachment within the knee joint compartment and is configured to be movable within the knee joint compartment during knee articulation.

## Patentansprüche

1. Unikompartimentelle Prothese (100) zur Implantation in ein Kniegelenkkompartiment zwischen einer Oberschenkelknorre und ihrem entsprechenden Schienbeinplateau, wobei die Prothese (100) umfasst:
einen Körper (102) mit einer in der Draufsicht im Allgemeinen elliptischen Form, wobei der Körper (102) eine untere Fläche (104), die mit einem Umriss versehen ist, der im Allgemeinen derselbe wie das Schienbeinplateau ist, und eine gegenüberliegende obere Fläche (106) aufweist, wobei der Körper (102) einen Umfangsrand (112) aufweist, der zwischen der oberen und unteren Fläche (104, 106) verläuft und eine erste Seite (114), eine zweite Seite (116) gegenüber der ersten Seite (114), ein erstes Ende (118) und ein zweites Ende (120) gegenüber dem ersten Ende (118) aufweist, **dadurch gekennzeichnet, dass** die obere Fläche (106) einen ersten, mittig angeordneten Abschnitt (107), der elliptisch geformt und im Allgemeinen eben ist, um die Konformität der oberen Fläche (106) mit der Oberschenkelknorre zu vermindern, damit eine übermäßige Bewegung des Körpers während der Bewegungen des Kniegelenks aufgrund von Erkrankungen der Oberschenkelknorre oder einer Bandinsuffizienz reduziert wird, sowie einen zweiten, konkaven Abschnitt (109) aufweist, der vom ersten Abschnitt (107) aus nach oben verläuft und einen Abstand zum Umfangsrand (112) um einen gesamten Umfang des ersten Abschnitts (107) herum aufweist, wobei der erste Abschnitt (107) einen anderen Krümmungsradius aufweist als der zweite Abschnitt (109), und wobei der zweite Abschnitt so eingerichtet ist, dass er im Allgemeinen mit einem Umriss der Oberschenkelknorre übereinstimmt, damit der Körper während der gesamten Kniebewegung auf die Oberschenkelknorre im Kniegelenkkompartiment zentriert bleibt.

2. Prothese nach Anspruch 1, wobei die untere Fläche (104) im Allgemeinen eben ist.

3. Prothese nach Anspruch 1, wobei die untere Fläche (104) mindestens eine von dort aus verlaufende Längsrippe (130) aufweist.

4. Prothese nach Anspruch 1, wobei der Körper (102) mindestens eine darin vorgesehene Öffnung (140) aufweist.

5. Prothese nach Anspruch 1, wobei der Körper (102) aus einem biokompatiblen Material aufgebaut ist, das aus der Gruppe bestehend aus Keramik, Metallen, Metalllegierungen, Hydrogelen, verstärkten und nicht verstärkten duroplastischen oder thermoplastischen Polymeren oder Verbundwerkstoffen daraus ausgewählt ist.

6. Prothese nach Anspruch 1, wobei der Körper (102) mindestens einen Abschnitt aus einem Material mit einem verhältnismäßig niedrigen Elastizitätsmodul aufweist, das aus der Gruppe bestehend aus verstärkten und nicht verstärkten elastomeren Polymeren, viskoelastischen Materialien und Hydrogelen ausgewählt ist.

7. Prothese nach Anspruch 1, wobei der Körper (102) ein Material aufweist, das in der Lage ist, lebende Zellen zu enthalten.

8. Prothese nach Anspruch 1, die ferner eine auf den Körper (102) aufgebrachte Oberflächenbeschichtung umfasst.

9. Prothese nach Anspruch 1, wobei der Körper (102) ein zugehöriges aktives Material aufweist.

10. Prothese nach Anspruch 1, wobei die obere Fläche (106) und die untere Fläche (104) einen derartigen Umriss aufweisen, dass sich die Prothese (100) im Kniegelenkkompartiment selbst zentriert.

11. Prothese nach Anspruch 1, wobei die erste oder die zweite Seite (114, 116) eine Einbuchtung (124) darin aufweist.

12. Prothese nach Anspruch 11, wobei der erste Abschnitt (107) an die Einbuchtung (124) angrenzt.

13. Prothese nach Anspruch 1, wobei eine erste Abmessung D durch das erste Ende (118) und das zweite Ende (120) definiert ist und eine zweite Abmessung F durch die erste Seite (114) und die zweite Seite (116) definiert ist, wobei die Abmessung F zwischen ungefähr 0,25D und ungefähr 1,5D liegt.

14. Prothese nach Anspruch 13, wobei eine Länge des ersten Abschnitts (107) zwischen ungefähr 0,5D und ungefähr 0,8D liegt.

15. Prothese nach Anspruch 13, wobei eine Breite des ersten Abschnitts (107) zwischen ungefähr 0,4F und 0,7F liegt.

16. Prothese nach Anspruch 1, wobei ein Schnitt quer über den Körper (102) vom ersten Ende (118) zum zweiten Ende (120) im Allgemeinen die Form eines negativen Meniskus aufweist.

17. Prothese nach Anspruch 1, wobei ein Rand des Körpers (102) im Durchschnitt dicker ist als ein mittiger Abschnitt des Körpers (102).

18. Prothese nach Anspruch 1, wobei der Körper (102) ein Material umfasst, das in der Lage ist, eventuelle Defekte in der Oberschenkelknorre und dem Schienbeinplateau zu überspannen, ohne sich im Wesentlichen in die Defekte hinein zu verformen.

19. Prothese nach Anspruch 1, wobei die Prothese (100) innerhalb des Kniegelenkkompartiments keine physikalische Verbindung aufweist und so eingerichtet ist, dass sie innerhalb des Kniegelenkkompartiments während der Kniebewegung beweglich ist.

## Revendications

1. Prothèse unicompartimentale (100) à implanter dans un compartiment d'articulation de genou entre le condyle fémoral et son plateau tibial correspondant, la prothèse (100) comprenant:
un corps (102) présentant une forme essentiellement elliptique en plan, le corps (102) présentant une surface inférieure (104) configurée avec un contour qui est essentiellement le même que celui du plateau tibial, et une surface supérieure opposée (106), le corps (102) comportant un bord périphérique (112) qui s'étend entre les surfaces inférieure et supérieure (104, 106) et qui présente un premier côté (114), un second côté (116) opposé au premier côté (114), une première extrémité (118) et une seconde extrémité (120) opposée à la première extrémité (118), **caractérisée en ce que** la surface supérieure (106) présente une première partie positionnée de façon centrale (107) qui est de forme elliptique et essentiellement plate en vue de réduire la conformité de la surface supérieure (106) au condyle fémoral dans le but de réduire un déplacement excessif du corps pendant un mouvement d'articulation de l'articulation du genou en raison d'une maladie dans le condyle fémoral ou d'une insuffisance ligamentaire, et une seconde partie concave (109) qui s'étend vers le haut à partir de la première partie (107) et qui est espacée du bord périphérique (112) autour de la totalité de la périphérie de la première partie (107), la première partie (107) présentant un rayon de courbure différent de celui de la seconde partie (109), et dans laquelle la seconde partie est configurée de manière à épouser essentiellement un contour du condyle fémoral de manière à permettre au corps de rester centré avec le condyle fémoral à l'intérieur du compartiment de l'articulation du genou d'un bout à l'autre du mouvement d'articulation du genou.

2. Prothèse selon la revendication 1, dans laquelle la surface inférieure (104) est essentiellement plate.

3. Prothèse selon la revendication 1, dans laquelle la surface inférieure (104) comprend au moins une quille (130) qui s'étend à partir de celle-ci.

4. Prothèse selon la revendication 1, dans laquelle le corps (102) comporte au moins une ouverture (140) pratiquée dans celui-ci.

5. Prothèse selon la revendication 1, dans laquelle le corps (102) est constitué d'un matériau biocompatible qui est sélectionné dans le groupe comprenant la céramique, des métaux, des alliages de métaux, des hydrogels, des polymères thermodurcis ou thermoplastiques renforcés et non renforcés, ou des composites de ceux-ci.

6. Prothèse selon la revendication 1, dans laquelle le corps (102) comprend au moins une partie d'un matériau à module d'élasticité relativement faible qui est sélectionné dans le groupe comprenant des polymères élastomères renforcés et non renforcés, des matériaux viscoélastiques et des hydrogels.

7. Prothèse selon la revendication 1, dans laquelle le corps (102) comprend un matériau capable de contenir des cellules vivantes.

8. Prothèse selon la revendication 1, comportant en outre un revêtement de surface qui est appliqué au corps (102) .

9. Prothèse selon la revendication 1, dans laquelle le corps (102) comprend un matériau actif associé à celle-ci.

10. Prothèse selon la revendication 1, dans laquelle la surface supérieure (106) et la surface inférieure (104) sont profilées de telle sorte que la prothèse (100) soit une prothèse à auto-centrage à l'intérieur du compartiment de l'articulation du genou.

11. Prothèse selon la revendication 1, dans laquelle un des premier et second côtés (114, 116) comporte une indentation (124) dans celui-ci.

12. Prothèse selon la revendication 11, dans laquelle la première partie (107) est adjacente à l'indentation (124) .

13. Prothèse selon la revendication 1, dans laquelle une première dimension D est définie par la première extrémité (118) et la seconde extrémité (120), et une seconde dimension F est définie par le premier côté (114) et le second côté (116), dans laquelle la dimension F est comprise entre environ 0,25 D et environ 1,5 D.

14. Prothèse selon la revendication 13, dans laquelle une longueur de la première partie (107) est comprise entre environ 0,5 D et environ 0,8 D.

15. Prothèse selon la revendication 13, dans laquelle une largeur de la première partie (107) est comprise entre environ 0,4 F et 0,7 F.

16. Prothèse selon la revendication 1, dans laquelle une section à travers le corps (102) partant de la première extrémité (118) jusqu'à la seconde extrémité (120) présente essentiellement la forme d'un ménisque divergent.

17. Prothèse selon la revendication 1, dans laquelle une périphérie du corps (102) présente en moyenne une épaisseur plus grande que celle d'une partie centrale du corps (102).

18. Prothèse selon la revendication 1, dans laquelle le corps (102) comprend un matériau capable de recouvrir tous les défauts potentiels dans le condyle fémoral et le plateau tibial sans se déformer sensiblement dans les défauts.

19. Prothèse selon la revendication 1, dans laquelle la prothèse (100) est exempte de fixation physique à l'intérieur du compartiment de l'articulation du genou et est configurée de manière à être déplaçable à l'intérieur du compartiment de l'articulation du genou pendant un mouvement d'articulation du genou.
